(19) Europäisches Patentamt
European Patent Office
Office européen des brevets

(11) EP 3 929 934 A1

(12) EUROPEAN PATENT APPLICATION
published in accordance with Art. 153(4) EPC

(43) Date of publication:
29.12.2021 Bulletin 2021/52

(51) Int Cl.:
$G16H\ 30/20^{(2018.01)}$

(21) Application number: 19916229.8

(86) International application number:
PCT/CN2019/101157

(22) Date of filing: 16.08.2019

(87) International publication number:
WO 2020/168695 (27.08.2020 Gazette 2020/35)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 22.02.2019 CN 201910132124

(71) Applicant: VR Doctor Medical Technology
(Shenzhen) Co., Ltd.
Shenzhen, Guangdong, 518035 (CN)

(72) Inventors:
• LEE, David Wei
Shenzhen, Guangdong 518035 (CN)
• LEE, Stewart Ping
Shenzhen, Guangdong 518035 (CN)

(74) Representative: Dargiewicz, Joanna
JD&P Patent Attorneys
Joanna Dargiewicz & Partners
Ul. Mysliborska 93A/50
03-185 Warszawa (PL)

(54) VRDS 4D MEDICAL IMAGE-BASED TUMOR AND BLOOD VESSEL AI PROCESSING METHOD AND PRODUCT

(57) Disclosed in embodiments of the present application are a method and a product for AI processing of tumor and blood vessel based on VRDS 4D medical images, the method is applied to a medical imaging apparatus, and the method includes determining a bitmap BMP data source according to a plurality of scanned images associated with a target organ of a target user; generating target medical image data according to the BMP data source; determining abnormal data in the target medical image data, a target tissue includes the target organ and a blood vessel; determining an association relationship between a tumor and a blood vessel of the target user according to a data set of the blood vessel and the abnormal data; performing 4D medical imaging according to the target medical image data, and outputting the association relationship between the tumor and the blood vessel. The embodiment of this application is facilitated to improve the accuracy and efficiency of the medical imaging apparatus in recognizing the association relationship between a tumor and a blood vessel.

| | |
|---|---|
| Medical imaging apparatus determining a bitmap BMP data source according to a plurality of scanned images associated with a target organ of a target user | S201 |
| The medical imaging apparatus generates target medical image data according to the BMP data source | S202 |
| The medical imaging apparatus determines the abnormal data in the target medical image data | S203 |
| The medical imaging apparatus determines an association relationship between a tumor of the target user and the blood vessel according to the data set of the blood vessel and the abnormal data | S204 |
| The medical imaging apparatus performs a 4D medical imaging according to the target medical image data, and outputs the association relationship between the tumor and the blood vessel | S205 |

Fig. 2

EP 3 929 934 A1

**Description**

## TECHNICAL FIELD

This application relates to the field of medical imaging apparatus, and in particular, to a method and a product for AI processing of tumor and blood vessel based on VRDS 4D medical images.

## BACKGROUND

In current, doctors still use watching to read continuous two-dimensional slice scanned images, for example, CT (Computed Tomography), MRI (Magnetic Resonance Imaging), DTI (Diffusion Tensor Imaging), PET (positron emission computed tomography), so as to perform judging and analyzing on the pathological tissue of the patients such as tumors. However, it is impossible to determine the association relationship between tumor and peripheral blood vessels only by looking directly at the two-dimensional slice data, which seriously affects the diagnosis of the doctors on the diseases. With rapid development of medical imaging technology, people put forward new demands for medical imaging.

## SUMMARY

Embodiments of this application provide a method and a product for AI processing of tumor and blood vessel based on VRDS 4D medical images, in order to improve the accuracy and efficiency of the medical imaging apparatus in recognizing of the association relationship between tumor and peripheral blood vessels

In a first aspect, embodiments of this application provide a method for AI processing of tumor and blood vessel based on VRDS 4D medical images, which is applied to medical imaging apparatus. The method includes:

determining a bitmap BMP data source according to a plurality of scanned images associated with a target organ of a target user;

generating target medical image data according to the BMP data source, wherein the target medical image data includes at least a data set of the target organ and a data set of a blood vessel around the target organ. The data set of the blood vessel includes a data set of an artery and/or a data set of a vein, and data of intersection positions of the artery and the vein is independent of each other, the data set of the target organ is a transfer function result of a cubic space between a surface of the target organ and a tissue structure inside the target organ, and the data set of the blood vessel is a transfer function result of a cubic space between a surface of the blood vessel and a tissue structure inside the blood vessel;

determining abnormal data in the target medical image data, wherein a target tissue includes the target organ and the blood vessel;

determining an association relationship between a tumor of the target user and the blood vessel according to the data set of the blood vessel and the abnormal data;

performing a 4D medical imaging according to the target medical image data, and outputting the association relationship between the tumor and the blood vessel.

In a second aspect, embodiments of this application provide an apparatus for processing tumor and blood vessel based on VRDS 4D medical images, which is applied to a medical imaging apparatus. The apparatus for processing tumor and blood vessel based on VRDS 4D medical image includes a processing unit and a communication unit, wherein,
the processing unit is configured to: determine a bitmap BMP data source according to a plurality of scanned images associated with a target organ of a target user; generate target medical image data according to the BMP data source, wherein the target medical image data includes at least a data set of the target organ and a data set of a blood vessel around the target organ. The data set of the blood vessel includes a data set of an artery and/or a data set of a vein, and data of intersection positions of the artery and the vein is independent of each other, the data set of the target organ is a transfer function result of a cubic space between a surface of the target organ and a tissue structure inside the target organ, and the data set of the blood vessel is a transfer function result of a cubic space between a surface of the blood vessel and a tissue structure inside the blood vessel; determine abnormal data in the target medical image data, wherein a target tissue includes the target organ and the blood vessel; determine an association relationship between the tumor of the target user and the blood vessel according to the data set of the blood vessel and the abnormal data; and perform 4D medical imaging according to the target medical image data and output the association relationship between the tumor and the blood vessel through the communication unit.

In a third aspect, embodiments of this application provide an medical imaging apparatus, the apparatus includes a processor, a memory, a communication interface, and one or more programs, wherein the above one or more programs are stored in the above memory and configured to be executed by the above processor, and the above programs include instructions for executing the steps in any method of the first aspect of the embodiment of this application.

In a fourth aspect, embodiments of this application provide a computer readable storage medium, wherein the above computer readable storage medium stores a computer program for electronic data exchange, wherein the above computer program causes a computer to execute some or all of the steps described in any methods of the first aspect of the embodiment of this application.

In a fifth aspect, embodiments of this application provide a computer program product, wherein the above computer program product includes a non-transitory computer-readable storage medium in which a computer program is stored, the above computer program is operable to make the computer execute some or all of the steps described in any methods of the first aspect of the embodiment of this application. The computer program product can be a software installation package.

As can be seen that in the embodiment of this application, first, the medical imaging apparatus determines a bitmap BMP data source according to a plurality of scanned images associated with a target organ of a target user; second, generates target medical image data according to the BMP data source; third, determines abnormal data in the target medical image data, a target tissue includes the target organ and a blood vessel; fourth, determines an association relationship between a tumor and a blood vessel of the target user according to a data set of the blood vessel and the abnormal data; finally, performs 4D medical imaging according to the target medical image data, and output the association relationship between tumor and blood vessel, wherein the target medical image data includes at least a data set of the target organ and a data set of a blood vessel around the target organ, the data set of the blood vessel includes a data set of an artery and/or a data set of a vein, and data of intersection positions of the artery and the vein is independent of each other, the data set of the target organ is a transfer function result of a cubic space between a surface of the target organ and a tissue structure inside the target organ, and the data set of the blood vessel is a transfer function result of a cubic space between a surface of the blood vessel and a tissue structure inside the blood vessel. It can be seen that the medical imaging apparatus in this application can obtain the association relationship between a tumor and a blood vessel by processing a plurality of scanned images, and output the association relationship, thus avoiding the situation of inaccurate observation based on human eyes, which is facilitated to improve the accuracy and efficiency of the medical imaging apparatus in recognizing the association relationship between a tumor and a blood vessel.

**BRIEF DESCRIPTION OF THE DRAWINGS**

In order to describe the technical solutions in the embodiments of this application or in the prior art more clearly, the following briefly describes the accompanying drawings required for describing the embodiments or the prior art, apparently, the accompanying drawings in the following description only show some embodiments of this application, and the ordinary skill person in the art may still derive other drawings from these accompanying drawings without creative efforts.

Fig. 1 is a schematic structural diagram of a medical image intelligent analysis and processing system based on VRDS 4D provided by an embodiment of this application;

Fig. 2 is a schematic flowchart of a method for AI processing of tumor and blood vessel based on VRDS 4D medical images provided by an embodiment of this application;

Fig. 3 is a schematic structural diagram of an medical imaging apparatus provided by an embodiment of this application;

Fig. 4 is a block diagram of functional units composition of an apparatus for AI processing of tumor and blood vessel based on VRDS 4D medical images provided by an embodiment of this application.

**DETAILED DESCRIPTION OF PREFERRED EMBODIMENTS**

In order to make person in the art better understand the solution of this application, the following clearly and completely describes the technical solutions in the embodiments of this application with reference to the accompanying drawings in the embodiments of this application, apparently, the described embodiments are only some but not all of the embodiments of this application. All other embodiments obtained by a person of ordinary skill in the art based on the embodiments of this application without creative efforts shall fall within the protection scope of this application.

The terms "first", "second", etc. in the specification and claims of this application and the above drawings are used to distinguish different objects, but not to describe a specific order. Furthermore, that term "including" and "having" and any variations thereof are intended to cover non-exclusive inclusion. For example, a process, method, system, product or device including a series of steps or units is not limited to the listed steps or units, but optionally further includes steps or units not listed, or optionally further includes other steps or units inherent to these processes, methods, products or devices.

Reference to an "embodiment" herein means that a particular feature, structure or characteristic described in connection with an embodiment may be included in at least one embodiment of this application. The appearances of the phrase in various places in the specification are not necessarily all referring to the same embodiment, nor are separate or alternative embodiments mutually exclusive of other embodiments. It is explicitly and implicitly to be understood by the person skilled in the art that the described embodiment may be combined with other embodiments.

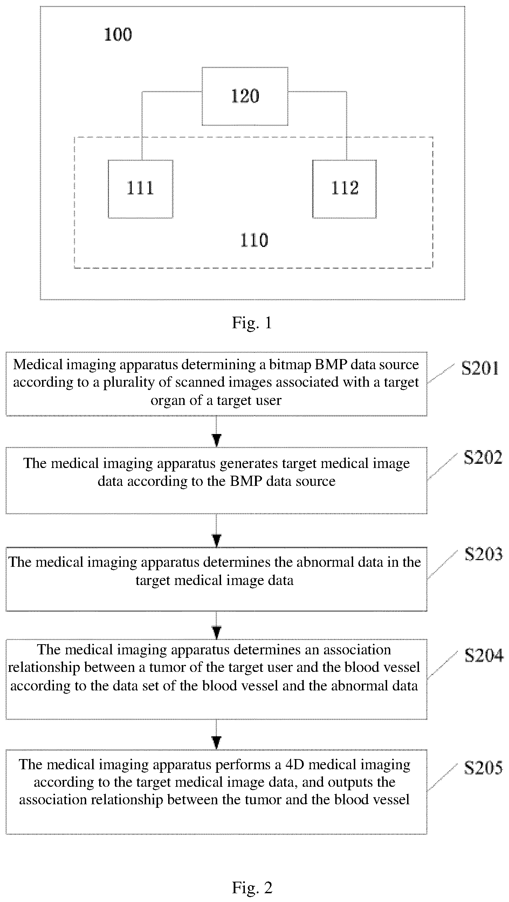

The medical imaging apparatuses related to the embodiments of this application refer to various instruments that use various different media as information carriers to reproduce the internal structure of the human body as images, and their image information has a corresponding relationship spatial and temporal distribution with the actual structure of the human body. "DICOM data" refers to the raw image file data collected by medical device and reflecting the internal structure features of human body, and can include information such as Computed Tomography (CT), Nuclear Magnetic Resonance (MRI), Diffusion Tensor Imaging (DTI), Positron Emission Computed Tomography (PET-CT), "image source" refers to Texture 2D/3D image volume data generated by parsing the raw DICOM data. "VRDS" refers to Virtual Reality Doctor system. Referring to Fig. 1, Fig. 1 is a schematic structural diagram of a medical image intelligent analysing and processing system 100 based on VRDS 4D provided by an embodiment of this application, the system 100 includes a medical imaging apparatus 110 and a network database 120, wherein the medical imaging apparatus 110 may include a local medical imaging apparatus 111 and/or a terminal medical imaging apparatus 112, the local medical imaging apparatus 111 or the terminal medical imaging apparatus 112 is configured to perform recognizing, positioning and four-dimensional volume drawing on the association relationship between human body tumor and blood vessel based on the raw DICOM data and the method for AI processing of tumor and blood vessel based on VRDS 4D medical image presented in the embodiment of this application, so as to achieve the four-dimensional stereo effect (the four-dimensional medical image specifically refers to the medical image including the internal spatial structure features and the external spatial structure features of the displayed tissue, the internal spatial structure features refer to slice data inside the tissues are not lost, that is, medical imaging apparatuses can present the internal constructions of tissues such as target organs and blood vessels, and the external spatial structure characteristics refer to the environmental features between tissues, including spatial position characteristics (including intersection, spacing and fusion) between tissues, such as edge structure characteristics of the intersection position between kidney and artery, etc.). Compared with the terminal medical imaging apparatus 112, the local medical imaging apparatus 111 can further configured to edit the image source data to form the transfer function results of the four-dimensional human body image, which can include the transfer function results of the surface of the internal organs of the human body and the tissue structure inside the internal organs of the human body, as well as the transfer function results of the cube space, such as the number, coordinates, colors, transparency and other information of the cube editing boxes and arc editing arrays required by the transfer function. The network database 120 can be, for example, a cloud server, etc.,

the network database 120 is configured to store the image source generated by parsing the raw DICOM data and the transfer function result of the four-dimensional human body image edited by the local medical imaging apparatus 111, the image source can come from a plurality of local medical imaging apparatuses 111 to achieve the interactive diagnosis of a plurality of doctors.

When a user performs a specific image displaying through the above medical imaging apparatus 110, the user can choose a display or a Head mounted Displays Set (HMDS) of virtual reality VR to display in combination with operation actions, which refer to the operation control of the four-dimensional human body image by the user through external intake device of the medical imaging apparatus, such as a mouse and a keyboard, so as to achieve human-computer interaction. The operation actions include at least one of the following: (1) changing the color and/or transparency of a specific organ/tissue, (2) positioning and scaling the view, (3) rotating the view to achieve multi-view 360-degree observation of the four-dimensional human image, (4) "entering" inside the organ of the human body to observe the internal construction, and the shearing effect rendering in real time, and (5) moving the view up and down.

The following describes a method for AI processing of tumor and blood vessel based on VRDS 4D medical images in detail.

Referring to Fig. 2, Fig. 2 is a schematic flowchart of a method for AI processing of tumor and blood vessel based on VRDS 4D medical images provided by an embodiment of this application, the method is applied to medical imaging apparatus as described in Fig. 1; as shown in the figure, the AI processing of tumor and blood vessel based on VRDS 4D medical images includes:

S201, determining, by a medical imaging apparatus, a bitmap BMP data source according to a plurality of scanned images associated with a target organ of a target user;

wherein the target organ can be, for example, a kidney. And the scanned image includes any one of the following: a CT image, an MRI image, a DTI image and a PET-CT image.

In this possible example, the medical imaging apparatus determines the bitmap BMP data source according to a plurality of scanned images associated with the target organ of the target user, including: the medical imaging apparatus acquires a plurality of scanned images collected by a medical device and reflecting internal structure features of human body of the target user; screens at least one scanned image including the target organ from the plurality of scanned images, and takes the at least one scanned image as medical digital imaging and communication DICOM data of the target user; parse the DICOM data to generate a image source of the target user, wherein the image source includes Texture 2D/3D image volume data; executes a first preset process for the image source to obtain the BMP data source, wherein the first preset process includes at least one of the following operations: VRDS limited contrast adaptive histogram equalization, mixed partial differential denoising and VRDS AI elastic deformation processing. Wherein, the VRDS limited contrast adaptive histogram equalization includes: regional noise ratio limiting and global contrast limiting; the local histogram of the image source is divided into a plurality of partitions, for each partition, the slope of the transform function is determined according to the slope of the cumulative histogram of the neighborhood of the partition, and the contrast amplification degree around the pixel value of the partition is determined according to the slope of the transform function, then, according to the contrast amplification degree, the limit clipping process is carried out to generate the distribution of effective histograms and the value of effective available neighborhood size, and these clipped partial histograms are uniformly distributed to other areas of the histogram.

Wherein the mixed partial differential denoising includes: different from Gaussian low-pass filtering (which weakens the high-frequency components of the image indiscriminately and blurs the edges of the image while denoising), the isophotes (including edges) formed by objects in the natural image should be smooth and unhindered curves, that is, the absolute value of curvature of these isophotes should be small enough, and when the image is polluted by noise, the local gray value of the image will fluctuate randomly, and it leads to the irregular oscillation of the isophotes and forms the isophotes with large local curvature, and according to this principle, a mixed partial differential denoising model is designed, which can protect the image edge and avoid the step effect in the smoothing process by VRDS AI curvature driving and VRDS AI high-order hybrid denoising.

Wherein the VRDS AI elastic deformation processing includes: superimposing positive and negative random distances on the original lattice to form a difference position matrix, and then forming a new lattice at the gray level of each difference position, so as to achieve the internal distortion of the image, and further performing rotation, distortion and translation operations etc. on the image.

It can be seen that in this example, the medical imaging apparatus obtains the BMP data source by processing the raw scanned image data, which increases the information amount of the raw data and increases the depth information, and finally obtains the data meeting the display requirements of the 4D medical image.

S202, the medical imaging apparatus generates target medical image data according to the BMP data source, wherein the target medical image data includes at least a data set of the target organ and a data set of a blood vessel around the target organ, the data set of the blood vessel includes a data set of an artery and/or a data set of a vein, and the data of intersection positions of the artery and the vein are independent of each other, the data set of the target organ is a transfer function result of a cubic space between a surface of the target organ and a tissue structure inside the target organ, and the data set of the blood vessel is a transfer function result of a cubic space between a surface of the blood vessel and the tissue structure inside the blood vessel.

In this possible example, the medical imaging apparatus generates the target medical image data according to the BMP data source, including: the medical imaging apparatus introduces the BMP data source into a preset VRDS medical network model to obtain a first medical image data, wherein the first medical image data includes the data set of the target organ and the data set of the blood vessel, and the data set of the blood vessel includes the fusion data of the intersection position of the artery and the vein; introduces the first medical image data into a preset cross blood vessel network model to obtain a second medical image data, wherein the second medical image data includes the data set of the target organ, the data set of the artery and the data set of the vein, and the first data in the data set of the artery and the second data in the data set of the vein are independent of each other; execute a first preset processing on the second medical image data to obtain target medical image data, wherein the first preset processing includes at least one of the following operations: 2D boundary optimization processing, 3D boundary optimization processing and data enhancement processing, and the target medical image data includes the data set of the target organ, the data set of the artery and the data set of the vein.Wherein the VRDS medical network model is provided with the transfer function of structural characteristics of target organs and the transfer function of structural characteristics of blood vessels, and the BMP data source obtains the first medical image data through the processing of the transfer function, and the cross blood vessel network model achieves the data separation of arteries and veins by the following operations: (1) extracting the fusion data of the intersection position; (2) for each fusion data, separating the fusion data based on a preset data separation algorithm to obtain artery boundary point data and vein boundary point data that are independent of each other; (3) integrating multiple artery boundary point data obtained after processing into first data and integrating multiple vein boundary point data obtained after processing into second data.

The 2D boundary optimization processing includes: acquiring low-resolution information and high-resolution information by sampling multiple times, wherein the low-resolution information can provide context semantic information of a segmentation target throughout the image, that is, features reflecting the relationship between the target and the environment, these features are used to judge the class of objects, and the high-resolution information is used to provide more fine features, such as gradients, for segmentation targets.

The 3D boundary optimization processing includes: 3D convolution, 3D maximum pooling and 3D upward convolution layer. The size of input data is al, a2 and a3, the number of channels is c, and the size of the filter is f, that is, the dimension of the filter is f*f*f*c, and the number of filters is n, so the final output of 3 dimensional convolution is:

$$(al\text{-}f\text{+}1)*(a2\text{-}f\text{+}l)*(a3\text{-}f\text{+}l)*n$$

Which has an analysis path and a synthesis path. In the analysis path, each layer includes two convolution kernels of 3*3*3, each of which follows an activation function (Relu), and then there is a maximum pooling of 2*2*2 on each dimension to merge the two step lengths. In the synthesis path, each layer is composed of 2*2*2 upward convolution, and the step length on each dimension is 2, next, two 3*3*3 convolutions, and then Relu. Shortcut connections from equal resolution layers in the analysis path then provide the basic high-resolution features of the synthetic path. In the last layer, 1*1*1 convolution reduces the number of output channels.

Wherein the data enhancement processing includes any one of the following: data enhancement based on arbitrary angle rotation, data enhancement based on histogram equalization, data enhancement based on white balance, data enhancement based on mirror operation, data enhancement based on random shearing and data enhancement based on simulating different illumination changes.

It can be seen that, in this example, the medical imaging apparatus can perform process on the BMP data source through the VRDS medical network model and the cross blood vessel network model and combine boundary optimization and data enhancement processing to obtain target image data, which solves the medical field problem that the traditional medical image cannot realize the whole separation of the segmented artery and vein, and improves the authenticity, comprehensiveness and refinement of the medical image display.

S203, the medical imaging apparatus determines abnormal data in the target medical image data, wherein a target tissue includes the target organ and the blood vessel.

S204, the medical imaging apparatus determines an association relationship between a tumor of the target user and the blood vessel according to the data set of the blood vessel and the abnormal data.

The association relationship includes any one of the following: fusion, proximity or keep away from.

In this possible example, the data set of the blood vessel includes the data set of the artery; the medical imaging apparatus determines the association relationship between the tumor of the target user and the blood vessel according to the data set of the blood vessel and the abnormal data including: the medical imaging apparatus determines the position area of the tumor of the target user according to the abnormal data; determines a spatial coordinate of each piece of data in the data set of the artery; and determines a position association relationship between the tumor and the artery according to the spatial coordinate of each piece of data and the position area, wherein the position association relationship includes fusion, proximity or keep away from.

Wherein the position area of the tumor is determined by the spatial coordinates of boundary data in the abnormal data, and if there are one or more spatial coordinates inside the space constrained by the boundary data in the spatial coordinates corresponding to the data set of an artery, it shows that there is a fusion relationship between the tumor and the artery, that is, a blood supply relationship in medicine; if there are one or more spatial coordinates in the boundary area constrained by the boundary data in the spatial coordinates corresponding to the data set of the artery, then there is a proximity relationship between tumor and artery; if there is no spatial coordinate in the space constrained by the boundary data in the corresponding spatial coordinates of the data set of the artery, there is a distant relationship between tumor and artery.

It can be seen that in this example, the medical imaging apparatus determines the association relationship between tumor and artery by analyzing the coordinate relationship between tumor and artery, because of the accuracy of the data, it is high accurate, convenient and efficient enough to determine the association relationship between tumor and artery.

In this example, The method further includes: the medical imaging apparatus determines type information of the tumor according to the position association relationship between the tumor and the artery, and outputs the type information. The type information of the tumor includes avascular tumors and vascular tumors, wherein avascular tumors refer to tumors that have not grown spiral tumor vessels (connected with arteries), while vascular tumors refer to tumors that have grown spiral tumor vessels (connected with arteries), for different types of tumors, doctors can selectively fuse tumor vessel blocking therapy to treat tumors.

It can be seen that, in this example, the medical imaging apparatus can determine the type of the tumor according to the position association relationship between the tumor and the artery, and output the information to guide doctors to perform targeted treatment, thus improving the comprehensiveness and functionality of tumor recognition.

In this possible example, the data set of the blood vessel includes the data set of the vein; the medical imaging apparatus determines the association relationship between the tumor of the target user and the blood vessel according to the data set of the blood vessel and the abnormal data including: the medical imaging apparatus detects whether the abnormal data and the data set of the vein containing the same data; if so, determines the position association relationship between the tumor of the target user and the vein according to the same data; if not, determines the position association relationship between the tumor of the target user and the vein is being far away.In specific implementation, if the same data includes boundary area data in abnormal data, the association relationship between tumor and vein is proximity, and if the same data includes internal area data of abnormal data, the position association relationship between tumor and vein is fusion.

Wherein the position area of the tumor is determined by the spatial coordinates of boundary data in the abnormal data, and if there are one or more spatial coordinates inside the space constrained by the boundary data in the spatial coordinates corresponding to the data set of a vein , it shows that there is a fusion relationship between the tumor and the vein, if there are one or more spatial coordinates in the boundary area constrained by the boundary data in the spatial coordinates corresponding to the data set of the vein, then there is a proximity relationship between tumor and vein, if there is no spatial coordinate in the space constrained by the boundary data in the corresponding spatial coordinates of the data set of the vein , there is a distant relationship between tumor and vein. The vein includes inferior vena cava.

It can be seen that in this example, the medical imaging apparatus determines the association relationship between tumor and vein by analyzing the coordinate relationship between tumor and vein, because of the accuracy of the data, it is high accurate , convenient and efficient enough to determine the association relationship between tumor and vein.

In this example, The method further includes: the medical imaging apparatus determines type information of the tumor according to the position association relationship between the tumor and the artery, and outputs the type information.

The type information of the tumor includes avascular tumors and vascular tumors, wherein avascular tumors refer to tumors that have not grown spiral tumor vessels (connected with veins), while vascular tumors refer to tumors that have grown spiral tumor vessels (connected with veins).For different types of tumors, doctors can selectively fuse tumor vessel blocking therapy to treat tumors.

It can be seen that, in this example, the medical imaging apparatus can determine the type of the tumor according to the position association relationship between the tumor and the vein, and output the information to guide doctors to perform targeted treatment, thus improving the comprehensiveness and functionality of tumor recognition.

In this possible example, the data set of the blood vessel includes the data set of the artery and the data set of the vein; the medical imaging apparatus determines the association relationship between the tumor of the target user and the blood vessel according to the data set of the blood vessel and the abnormal data including: the medical imaging apparatus compares the data set of the artery and the data set of the vein with the abnormal data respectively, and determines a first position relationship between the tumor of the target user and the artery and a second position relationship between the tumor and the vein; determines a blood supply relationship between the tumor and the blood vessel according to the first position relationship and the second position relationship.

Wherein first position relationship and second position relationship can comprehensively and accurately determine the blood supply relationship between tumor and all levels of blood vessels, thus providing information support.

It can be seen that in this example, the medical imaging apparatus can comprehensively analyze the first position relationship between tumor and artery and the second position relationship between tumor and vein, and determine the blood supply relationship of tumor according to the first position relationship and the second position relationship, thus improving the comprehensiveness and accuracy of tumor diagnosis.

In this example, the method further includes that the medical imaging apparatus determines a danger degree of the tumor according to the blood supply relationship, and outputs the danger degree.

The danger degree is determined by the blood supply relationship of tumor. According to the danger degree, the medical imaging apparatus can make a recommended tumor treatment scheme for doctors reference.

It can be seen that in this example, the medical imaging apparatus can determine the danger degree of tumor according to the blood supply relationship, and recommend the treatment scheme, thus improving the intelligence and comprehensiveness of tumor diagnosis.

S205, the medical imaging apparatus performs a 4D medical imaging according to the target medical image data, and outputs the association relationship between the tumor and the blood vessel.

Wherein the 4D medical imaging refers to presenting a the four-dimensional medical image.

In one possible example, the medical imaging apparatus preforms the 4D medical imaging according to the target medical image data, including: the medical imaging apparatus screens enhanced data with a quality score greater than a preset score from the target medical image data as VRDS 4D imaging data; and performs 4D medical imaging according to the VRDS 4D imaging data.

Wherein the quality score can be comprehensively evaluated from the following dimensions: average gradient, information entropy, visual information fidelity, peak signal-to-noise ratio PSNR, structural similarity SSIM, mean square error MSE, etc, specific reference can be made to common image quality score algorithms in the image field, which is not described details for brevity.

It can be seen that, in this example, the medical imaging apparatus further performs data screening through the quality score, thus improving the imaging effect.

As can be seen that in the embodiment of this application, firstly, the medical imaging apparatus determines a bitmap BMP data source according to a plurality of scanned images associated with a target organ of a target user; secondly, generates target medical image data according to the BMP data source; third, determines abnormal data in the target medical image data, a target tissue includes the target organ and a blood vessel; fourth, determines an association relationship between a tumor and a blood vessel of the target user according to a data set of the blood vessel and the abnormal data, finally, performs 4D medical imaging according to the target medical image data, and outputs the association relationship between tumor and blood vessel. Wherein the target medical image data includes at least a data set of the target organ and a data set of a blood vessel around the target organ, the data set of the blood vessel includes a data set of an artery and/or a data set of a vein, and data of intersection positions of the artery and the vein is independent of each other, the data set of the target organ is a transfer function result of a cubic space between a surface of the target organ and a tissue structure inside the target organ, and the data set of the blood vessel is a transfer function result of a cubic space between a surface of the blood vessel and a tissue structure inside the blood vessel. It can be seen that the medical imaging apparatus in this application can obtain the association relationship between a tumor and a blood vessel by processing a plurality of scanned images, and output the association relationship, thus avoiding the situation of inaccurate observation based on human eyes, which is beneficial to improve the accuracy and efficiency of the medical imaging apparatus in recognizing the association relationship between a tumor and a blood vessel.

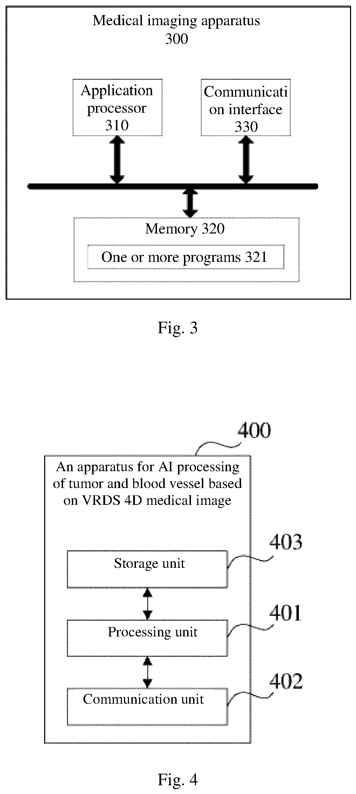

Consistent with the embodiments shown in Fig. 2, referring to Fig. 3, Fig. 3 is a schematic structural diagram of a medical imaging apparatus 300 provided by an embodiment of this application, as shown in the Fig, the medical imaging apparatus 300 includes a processor 310, a memory 320, a communication interface 330 and one or more programs 321, wherein the one or more programs 321 are stored in the above memory 320 and configured to be executed by the above processor 310, and the one or more programs 321 include instructions for executing the following steps:

determining a bitmap BMP data source according to a plurality of scanned images associated with a target organ of a target user; generating target medical image data according to the BMP data source, wherein the target medical image data includes at least a data set of the target organ and a data set of a blood vessel around the target organ, the data set of the blood vessel includes a data set of an artery and/or a data set of a vein, and data of intersection positions of the artery and the vein is independent of each other, the data set of the target organ is a transfer function result of a cubic space between a surface of the target organ and a tissue structure inside the target organ, and the data set of the blood vessel is a transfer function result of a cubic space between a surface of the blood vessel and a tissue structure inside the blood vessel; determining abnormal data in the target medical image data, wherein a target tissue includes the target organ and the blood vessel; determining an association relationship between the tumor of the target user and the blood vessel according to the data set of the blood vessel and the abnormal data; and performing 4D medical imaging according to the target medical image data and outputting the association relationship between the tumor and the blood vessel.

As can be seen that in the embodiment of this application, firstly, the medical imaging apparatus determines a bitmap BMP data source according to a plurality of scanned images associated with a target organ of a target user; secondly, generates target medical image data according to the BMP data source; third, determines abnormal data in the target medical image data, a target tissue includes the target organ and a blood vessel; fourth, determines an association relationship between a tumor and a blood vessel of the target user according to a data set of the blood vessel and the abnormal data; finally, performs 4D medical imaging according to the target medical image data, and outputting the association relationship between tumor and blood vessel. Wherein the target medical image data includes at least a data set of the target organ and a data set of a blood vessel around the target organ, the data set of the blood vessel includes a data set of an artery and/or a data set of a vein, and data of intersection positions of the artery and the vein is independent of each other, the data set of the target organ is a transfer function result of a cubic space between a surface of the target organ and a tissue structure inside the target organ, and the data set of the blood vessel is a transfer function result of a cubic space between a surface of the blood vessel and a tissue structure inside the blood vessel. It can be seen that the medical imaging apparatus in this application can obtain the association relationship between a tumor and a blood vessel by processing a plurality of scanned images, and output the association relationship, thus avoiding the situation of inaccurate observation based on human eyes, which is facilitated to improve the accuracy and efficiency of the medical imaging apparatus in recognizing the association relationship between a tumor and a blood vessel.

In one possible example, the data set of the blood vessel includes the data set of the artery; in the aspect of the determining of the association relationship between the tumor of the target user and the blood vessel according to the data set of the blood vessel and the abnormal data, the instructions in the program are specifically configured to perform the following operation: determining the position area of the tumor of the target user according to the abnormal data; and determining a spatial coordinate of each data in the data set of the artery; and determining a position association relationship between the tumor and the artery according to the spatial coordinate of each data and the position area, wherein the position association relationship includes fusion, proximity or keep away from.

In one possible example, the program also includes instructions configured to perform the following operation: determining type information of the tumor according to the position association relationship between the tumor and the artery; and outputting the type information.

In one possible example, the data set of the blood vessel includes the data set of the vein; in the aspect of the determining of the association relationship between the tumor of the target user and the blood vessel according to the data set of the blood vessel and the abnormal data, the program also includes instructions configured to perform the following operation: detecting whether the abnormal data and the data set of the vein include the same data; and if so, determining the position association relationship between the tumor of the target user and the vein according to the same data; and if not, determining the position association relationship between the tumor of the target user and the vein is being far away.In one possible example, the program also includes instructions configured to perform the following operation: determining type information of the tumor according to the position association relationship between the tumor and the artery; and outputting the type information.

In one possible example, the data set of the blood vessel includes the data set of the artery and the data set of the vein; in the aspect of the determining of the association relationship between the tumor of the target user and the blood vessel according to the data set of the blood vessel and the abnormal data, the instructions in the program are specifically configured to perform the following operation: comparing the data set of the artery and the data set of the vein with the abnormal data respectively, and determining a first position relationship between the tumor of the target user and the artery and a second position relationship between the tumor and the vein; and determining a blood supply relationship between the tumor and the blood vessel according to the first position relationship and the second position relationship. In one possible example, the program also includes instructions configured to perform the following operation: determining a danger degree of the tumor according to the blood supply relationship; and outputting danger degree.

The above mainly introduces the scheme of the embodiment of this application from the perspective of the execution process on the method side. It can be understood that in order to achieve the above functions, the medical imaging apparatus includes corresponding hardware structures and/or software modules for performing various functions. It should be easy for person of skill in the art aware that, in combination with the units and algorithmic steps of the examples described in the embodiments provided herein, this application can be implemented in the form of hardware or a combination of hardware and computer software. Whether the functions are performed by hardware or computer software driving hardware depends on particular applications and design constraint conditions of the technical solutions. A person skilled in the art may use different methods to implement the described functions for each particular application, but it should not be considered that the implementation goes beyond the scope of this application.

The embodiment of this application can divide the medical imaging apparatus into functional units according to the above method example, for example, individual functional unit can be divided corresponding to individual function, or two or more functions can be integrated into one processing unit. The integrated units can be implemented in the form of hardware, and can also be implemented in the form of a software functional unit. It should be noted that the division of units in the embodiment of this application is schematic, which is only a logical function division, and there may be another division mode in actual implementation. Fig. 4 is a block diagram of functional units composition of an apparatus 400 for processing tumor and blood vessel based on VRDS 4D medical images involved in the embodiment of this application. The apparatus 400 for AI processing of tumor and blood vessel based on VRDS 4D medical images is applied to a medical imaging apparatus, the apparatus 400 for AI processing of tumor and blood vessel based on VRDS 4D medical image includes a processing unit 401 and a communication unit 402.

The processing unit 401 is configured to determine a bitmap BMP data source according to a plurality of scanned images associated with a target organ of a target user; generate target medical image data according to the BMP data source, wherein the target medical image data includes at least a data set of the target organ and a data set of a blood vessel around the target organ, the data set of the blood vessel includes a data set of an artery and/or a data set of a vein, and data of intersection positions of the artery and the vein is independent of each other, the data set of the target organ is a transfer function result of a cubic space between a surface of the target organ and a tissue structure inside the target organ, and the data set of the blood vessel is a transfer function result of a cubic space between a surface of the blood vessel and a tissue structure inside the blood vessel; determine abnormal data in the target medical image data, wherein a target tissue includes the target organ and the blood vessel; determine an association relationship between the tumor of the target user and the blood vessel according to the data set of the blood vessel and the abnormal data; and perform 4D medical imaging according to the target medical image data and outputting the association relationship between the tumor and the blood vessel through the communication unit.

The processing apparatus 400 further includes a storage unit 403, wherein the processing unit 401 can be a processor, the communication unit 402 can be a communication interface, and the storage unit 403 can be a memory.

As can be seen that in the embodiment of this application, firstly, the medical imaging apparatus determines a bitmap BMP data source according to a plurality of scanned images associated with a target organ of a target user; secondly, generates target medical image data according to the BMP data source; third, determines abnormal data in the target medical image data, a target tissue includes the target organ and a blood vessel; fourth, determines an association relationship between a tumor and a blood vessel of the target user according to a data set of the blood vessel and the abnormal data; finally, performs 4D medical imaging according to the target medical image data, and outputs the association relationship between tumor and blood vessel. Wherein the target medical image data includes at least a data set of the target organ and a data set of a blood vessel around the target organ, the data set of the blood vessel includes a data set of an artery and/or a data set of a vein, and data of intersection positions of the artery and the vein is independent of each other, the data set of the target organ is a transfer function result of a cubic space between a surface of the target organ and a tissue structure inside the target organ, and the data set of the blood vessel is a transfer function result of a cubic space between a surface of the blood vessel and a tissue structure inside the blood vessel. It can be seen that the medical imaging apparatus in this application can obtain the association relationship between a tumor and a blood vessel by processing a plurality of scanned images, and output the association relationship, thus avoiding the situation of inaccurate observation based on human eyes, which is beneficial to improve the accuracy and efficiency of the medical imaging apparatus in recognizing the association relationship between a tumor and a blood vessel.

In one possible example, the data set of the blood vessel includes the data set of the artery; in the aspect of the determining of the association relationship between the tumor of the target user and the blood vessel according to the data set of the blood vessel and the abnormal data, the processing unit 401 is specifically configured to: determine the position area of the tumor of the target user according to the abnormal data; and determine a spatial coordinate of each data in the data set of the artery; and determine a position association relationship between the tumor and the artery according to the spatial coordinate of each data and the position area, wherein the position association relationship includes fusion, proximity or keep away from.

In one possible example, the processing unit 401 is further configured to: determine type information of the tumor according to the position association relationship between the tumor and the artery; and output the type information.

In one possible example, the data set of the blood vessel includes the data set of the vein; in the aspect of the determining of the association relationship between the tumor of the target user and the blood vessel according to the data set of the blood vessel and the abnormal data, the processing unit 401 is specifically configured to detect whether the abnormal data and the data set of the vein include the same data; and if so, determine the position association relationship between the tumor of the target user and the vein according to the same data; and if not, determine the position association relationship between the tumor of the target user and the vein is being far away.

In one possible example, the processing unit 401 is further configured to determine type information of the tumor according to the position association relationship between the tumor and the artery and output the type information.

In one possible example, the data set of the blood vessel includes the data set of the artery and the data set of the vein; in the aspect of the determining of the association relationship between the tumor of the target user and the blood vessel according to the data set of the blood vessel and the abnormal data. The processing unit 401 is specifically configured to compare the data set of the artery and the data set of the vein with the abnormal data respectively, and determine a first position relationship between the tumor of the target user and the artery and a second position relationship between the tumor and the vein, and determine a blood supply relationship between the tumor and the blood vessel according to the first position relationship and the second position relationship.

In one possible example, the processing unit 401 is specifically configured to determine a danger degree of the tumor according to the blood supply relationship, and output danger degree.

Embodiments of this application further provide a computer storage medium, wherein the computer storage medium stores a computer program for electronic data exchange, the computer program causes a computer to execute part or all of the steps of any method as recorded in the above method embodiment, and the above computer includes a medical imaging apparatus.

Embodiments of this application further provide a computer program product, the above computer program product includes a non-transitory computer-readable storage medium in which a computer program is stored, the above computer program is operable to cause a computer to execute part or all of the steps of any method as recorded in the above method embodiments. The computer program product can be a software installation package, and the above computer includes a medical imaging apparatus.

It should be noted that, for brevity of description, the foregoing method embodiments are described as a series of movement combinations. However, a person skilled in the art should learn that this application is not limited by the movement sequence, because according to this application, some steps can be performed in other order or simultaneously. Secondly, it also should be known by those skilled in the art that the embodiments described in the specification are preferred embodiments and the involved actions and modules are not the must of the present application necessarily.

In the above embodiments, the descriptions of individual embodiment have their own emphasis, for those parts that are not detailed in one embodiment, please refer to the relevant descriptions of other embodiments.

In the several embodiments provided in this application, it should be understood that the disclosed apparatus may be implemented in other manners. For example, the described apparatus embodiment is merely an example. For example, the above unit division is merely logical function division and may be other division in actual implementation. For example, a plurality of units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electronic, or other forms. The above units described as separate parts may or may not be physically separate, and parts displayed as units may or may not be physical units, may be located in one position, or may be distributed on a plurality of network units. It can select some or all of units to achieve the objective of the solution of the present embodiment based on actual requirements.

In addition, functional units in the embodiments of this application may be integrated into one processing unit, or each of the units may exist alone physically, or two or more units are integrated into one unit. The integrated units can be implemented in the form of hardware, and can also be implemented in the form of a software functional unit.

When the above integrated units are implemented in the form of a software functional unit and sold or used as an independent product, the functions may be stored in a computer readable memory. Based on such an understanding, the technical solutions of this application essentially, or the part contributing to the prior art, all or some of the technical solutions may be implemented in a form of a software product. The computer software product is stored in a memory, and includes several instructions for instructing a computer device (which may be a personal computer, a server, or a network device) to perform all or some of the steps of the above methods described in the embodiments of this application. The above memory may include any medium that can store program code, such as a USB flash disk, a read-only memory (ROM), a random access memory (RAM) removable hard disk, a magnetic disk, or an optical disc.

Those skilled in the art may understand that all or some of the steps in various methods of the above embodiments can be completed by instructing related hardware through programs, which can be stored in a computer readable memory, which may include flash disks, Read-Only Memory (ROM) random access memory (RAM) disks or optical disks, etc.

The embodiments of this application are described in detail above, and the principles and implementation of this application are explained by specific examples. The above embodiments are only used to help understand the method and its core ideas of this application; at the same time, according to the idea of this application, there will be some changes in the specific implementation and application scope for a person of skill in the art, to sum up, the contents of this specification should not be construed as limitations of this application.

**Claims**

1. A method for AI processing of tumor and blood vessel based on Virtual Reality Doctor system (VRDS) 4D medical images, wherein the method is applied to a medical imaging apparatus; and **characterized in that** the method comprises:

   determining a bitmap (BMP) data source according to a plurality of scanned images associated with a target organ of a target user;
   generating target medical image data according to the BMP data source, wherein the target medical image data comprises at least a data set of the target organ and a data set of a blood vessel around the target organ, the data set of the blood vessel comprises a data set of an artery and/or a data set of a vein, and data of intersection positions of the artery and the vein is independent of each other, the data set of the target organ is a transfer function result of a cubic space between a surface of the target organ and a tissue structure inside the target organ, and the data set of the blood vessel is a transfer function result of a cubic space between a surface of the blood vessel and a tissue structure inside the blood vessel;
   determining abnormal data in the target medical image data, wherein a target tissue comprises the target organ and the blood vessel;
   determining an association relationship between a tumor of the target user and the blood vessel according to the data set of the blood vessel and the abnormal data;
   performing a 4D medical imaging according to the target medical image data, and outputting the association relationship between the tumor and the blood vessel.

**2.** The method according to claim 1, **characterized in that** the data set of the blood vessel comprises the data set of the artery; the determining of the association relationship between the tumor of the target user and the blood vessel according to the data set of the blood vessel and the abnormal data comprises:

    determining position area of the tumor of the target user according to the abnormal data; determining a spatial coordinate of each data in the data set of the artery; determining the position association relationship between the tumor and the artery according to the spatial coordinate of each data and the position area, the position association relationship comprises fusion, proximity or being far away.

**3.** The method according to claim 2, **characterized in that** the method further comprises:

    determining type information of the tumor according to the position association relationship between the tumor and the artery; outputting the type information.

**4.** The method according to claim 1, **characterized in that** the data set of the blood vessel comprises the data set of the vein; the determining of the association relationship between the tumor of the target user and the blood vessel according to the data set of the blood vessel and the abnormal data comprises:

    detecting whether the abnormal data and the data set of the vein comprise the same data; if so, determining the position association relationship between the tumor of the target user and the vein according to the same data; if not, determining the position association relationship between the tumor of the target user and the vein is being far away.

**5.** The method according to claim 4, **characterized in that** the method further comprises: determining type information of the tumor according to the position association relationship between the tumor and the artery; outputting the type information.

**6.** The method according to claim 1, **characterized in that** the data set of the blood vessel comprises the data set of the artery and the vein; the determining of the association relationship between the tumor of the target user and the blood vessel according to the data set of the blood vessel and the abnormal data comprises:

comparing the data set of the artery and the data set of the vein with the abnormal data respectively, and determining a first position relationship between the tumor of the target user and the artery and a second position relationship between the tumor and the vein; determining a blood supply relationship between the tumor and the blood vessel according to the first position relationship and the second position relationship.

**7.** The method according to claim 6, **characterized in that** the method further comprises: determining a danger degree of the tumor according to the blood supply relationship; outputting the danger degree.

**8.** The method according to any one of claims 2 to 7, **characterized in that** the type information of the tumor comprises avascular tumor and vascular tumor.

**9.** The method according to claim 1, **characterized in that** the performing of the 4D medical imaging according to the target medical image data comprises:

    screening enhanced data with a quality score greater than a preset score from the target medical image data as VRDS 4D imaging data; performing 4D medical imaging according to the VRDS 4D imaging data.

**10.** An apparatus for AI processing of tumor and blood vessel based on VRDS 4D medical images, wherein the apparatus is applied to a medical imaging apparatus; **characterized in that** the apparatus for AI processing of tumor and blood vessel based on VRDS 4D medical image comprises a processing unit and a communication unit, wherein the processing unit is configured to: determine a bitmap BMP data source according to a plurality of scanned images associated with a target organ of a target user; generate target medical image data according to the BMP data source, wherein the target medical image data comprises at least a data set of the target organ and a data set of a blood vessel around the target organ, the data set of the blood vessel comprises a data set of an artery and/or a data set of a vein, and data of intersection positions of the artery and the vein is independent of each other, the data set of the target organ is a transfer function result of a cubic space between a surface of the target organ and a tissue structure inside the target organ, and the data set of the blood vessel is a transfer function result of a cubic space between a surface of the blood vessel and a tissue structure inside the blood vessel; determine abnormal data in the target medical image data, wherein a target tissue comprises the target organ and the blood vessel;

determine an association relationship between the tumor of the target user and the blood vessel according to the data set of the blood vessel and the abnormal data; and perform 4D medical imaging according to the target medical image data and outputting the association relationship between the tumor and the blood vessel through the communication unit.

11. The apparatus according to claim 10, **characterized in that** the data set of the blood vessel comprises the data set of the artery; in the aspect of the determining of the association relationship between the tumor of the target user and the blood vessel according to the data set of the blood vessel and the abnormal data, the processing unit is specifically configured to: determine the position area of the tumor of the target user according to the abnormal data; determine a spatial coordinate of each piece of data in the data set of the artery; and determine a position association relationship between the tumor and the artery according to the spatial coordinate of each piece of data and the position area, wherein the position association relationship comprises fusion, proximity or keep away from.

12. The apparatus according to claim 11, **characterized in that** the processing unit is further specifically configured to: determine type information of the tumor according to the position association relationship between the tumor and the artery; and output the type information.

13. The apparatus according to claim 10, **characterized in that** the data set of the blood vessel comprises the data set of the vein; in the aspect of the determining of the association relationship between the tumor of the target user and the blood vessel according to the data set of the blood vessel and the abnormal data, the processing unit is specifically configured to: detect whether the abnormal data and the data set of the vein comprise the same data; if so, determine the position association relationship between the tumor of the target user and the vein according to the same data; if not, determine the position association relationship between the tumor of the target user and the vein is being far away.

14. The apparatus according to claim 13, **characterized in that** the processing unit is further specifically configured to: determine the type information of the tumor according to the position association relationship between the tumor and the artery; and output the type information.

15. The apparatus according to claim 10, **characterized in that** the data set of the blood vessel comprises the data set of the artery and the data set of the vein;

in the aspect of the determining of the association relationship between the tumor of the target user and the blood vessel according to the data set of the blood vessel and the abnormal data, the processing unit is specifically configured to: compare the data set of the artery and the data set of the vein with the abnormal data respectively, and determine a first position relationship between the tumor of the target user and the artery and a second position relationship between the tumor and the vein; determine a blood supply relationship between the tumor and the blood vessel according to the first position relationship and the second position relationship.

16. The apparatus according to claim 15, **characterized in that** the processing unit is further specifically configured to: determine a danger degree of the tumor according to the blood supply relationship; and output the danger degree.

17. The apparatus according to any one of claims 10 to 16, **characterized in that** the type information of the tumor comprises avascular tumor and vascular tumor.

18. The apparatus according to claim 10, **characterized in that** in the aspect of the preforming of the 4D medical imaging according to the target medical image data, the communication unit is specifically configured to: screen enhanced data with a quality score greater than a preset score from the target medical image data as VRDS 4D imaging data; and perform 4D medical imaging according to the VRDS 4D imaging data.

19. A medical imaging apparatus, **characterized in that** the apparatus comprises a processor, a memory, a communication interface, and one or more programs, the one or more programs are stored in the memory and configured to be executed by the processor, and the programs comprise instructions for executing the steps in the method according to any one of claims 1-9.

20. A computer readable storage medium, **characterized in that** the computer readable storage medium stores a computer program for electronic data exchange, wherein the computer program causes a computer to execute the method according to any one of claims 1-9.

100

120

111

112

110

Fig. 1

| Medical imaging apparatus determining a bitmap BMP data source according to a plurality of scanned images associated with a target organ of a target user | S201 |

| The medical imaging apparatus generates target medical image data according to the BMP data source | S202 |

| The medical imaging apparatus determines the abnormal data in the target medical image data | S203 |

| The medical imaging apparatus determines an association relationship between a tumor of the target user and the blood vessel according to the data set of the blood vessel and the abnormal data | S204 |

| The medical imaging apparatus performs a 4D medical imaging according to the target medical image data, and outputs the association relationship between the tumor and the blood vessel | S205 |

Fig. 2

Medical imaging apparatus
300

Application
processor
310

Communicati
on interface
330

Memory 320

One or more programs 321

Fig. 3

400

An apparatus for AI processing
of tumor and blood vessel based
on VRDS 4D medical image

403

Storage unit

401

Processing unit

402

Communication unit

Fig. 4

# EP 3 929 934 A1

<table>
<tr><td colspan="2">INTERNATIONAL SEARCH REPORT</td><td>International application No.</td></tr>
<tr><td colspan="2"></td><td>**PCT/CN2019/101157**</td></tr>
</table>

**A.    CLASSIFICATION OF SUBJECT MATTER**

G16H 30/20(2018.01)i

According to International Patent Classification (IPC) or to both national classification and IPC

**B.    FIELDS SEARCHED**

Minimum documentation searched (classification system followed by classification symbols)

G16H; G06F

Documentation searched other than minimum documentation to the extent that such documents are included in the fields searched

Electronic data base consulted during the international search (name of data base and, where practicable, search terms used)

CNPAT, CNKI, EPODOC, WPI, IEEE: 器官, 位图, VRDS, 血管, 肿瘤, 动脉, 静脉, organic, bitmap, blood vessel, knub, tumour, artery, vein

**C.    DOCUMENTS CONSIDERED TO BE RELEVANT**

| Category* | Citation of document, with indication, where appropriate, of the relevant passages | Relevant to claim No. |
|---|---|---|
| A | CN 105631930 A (GUANGZHOU JUPU TECHNOLOGY CO.,, LTD.) 01 June 2016 (2016-06-01)<br>    description, paragraphs [0036]-[0044] | 1-20 |
| A | CN 102938027 A (HEBEI UNIVERSITY) 20 February 2013 (2013-02-20)<br>    entire document | 1-20 |
| A | US 2017071671 A1 (SIEMENS HEALTHCARE GMBH) 16 March 2017 (2017-03-16)<br>    entire document | 1-20 |

☐ Further documents are listed in the continuation of Box C.          ☑ See patent family annex.

| | |
|---|---|
| *    Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **10 October 2019** | **30 October 2019** |

| Name and mailing address of the ISA/CN | Authorized officer |
|---|---|
| **China National Intellectual Property Administration (ISA/ CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2019/101157**

| Patent document cited in search report | | | Publication date (day/month/year) | Patent family member(s) | | | Publication date (day/month/year) |
|---|---|---|---|---|---|---|---|
| CN | 105631930 | A | 01 June 2016 | None | | | |
| CN | 102938027 | A | 20 February 2013 | None | | | |
| US | 2017071671 | A1 | 16 March 2017 | EP | 3142033 | A1 | 15 March 2017 |
| | | | | CN | 106529117 | A | 22 March 2017 |

Form PCT/ISA/210 (patent family annex) (January 2015)